# EUROPEAN PATENT SPECIFICATION

(11) **EP 3 834 639 B1**
(45) Date of publication and mention of the grant of the patent: **02.08.2023**
(21) Application number: 19216247.7
(22) Date of filing: 13.12.2019
(51) Int. Cl.: A24F 40/10, A24F 40/50, A24F 40/53, A24F 40/60, A24F 40/65, H04L 67/125, A61M 15/06, A61M 11/04, A61M 16/00

(54) **AEROSOL DELIVERY SYSTEM**
AEROSOLABGABESYSTEM
SYSTÈME D'ADMINISTRATION D'AÉROSOL

(43) Date of publication of application: 16.06.2021
(73) Proprietor: Imperial Tobacco Limited, Bristol, BS3 2LL (GB)
(72) Inventor: TALBOT, Oliver, Speke, Liverpool L24 9HP (GB)
(74) Representative: Mewburn Ellis LLP

(56) References cited:
- EP-A1- 3 378 339
- WO-A1-2019/173923
- WO-A1-2019/186147

## Description

### Technical field

The present disclosure relates to an aerosol delivery system such as a smoking substitute system, and a method performed by the aerosol delivery system.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful by-products. There have been proposed various smoking substitute systems in order to avoid the smoking of tobacco.

Such smoking substitute systems can form part of nicotine replacement therapies aimed at people who wish to stop smoking and overcome a dependence on nicotine.

Smoking substitute systems, which may also be known as electronic nicotine delivery systems, may comprise electronic systems that permit a user to simulate the act of smoking by producing an aerosol, also referred to as a "vapour", which is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. Some smoking substitute systems are designed to resemble a traditional cigarette and are cylindrical in form with a mouthpiece at one end. Other smoking substitute systems do not generally resemble a cigarette (for example, the smoking substitute device may have a generally box-like form).

There are a number of different categories of smoking substitute systems, each utilising a different smoking substitute approach. A smoking substitute approach corresponds to the manner in which the substitute system operates for a user.

One approach for a smoking substitute system is the so-called "vaping" approach, in which a vaporisable liquid, typically referred to (and referred to herein) as "e-liquid", is heated by a heater to produce an aerosol vapour which is inhaled by a user. An e-liquid typically includes a base liquid as well as nicotine and/or flavourings. The resulting vapour therefore typically contains nicotine and/or flavourings. The base liquid may include propylene glycol and/or vegetable glycerine.

Atypical vaping smoking substitute system includes a mouthpiece, a power source (typically a battery), a tank or liquid reservoir for containing e-liquid, as well as a heater. In use, electrical energy is supplied from the power source to the heater, which heats the e-liquid to produce an aerosol (or "vapour") which is inhaled by a user through the mouthpiece.

Vaping smoking substitute systems can be configured in a variety of ways. For example, there are "closed system" vaping smoking substitute systems which typically have a heater and a sealed tank which is pre-filled with e-liquid and is not intended to be refilled by an end user. One subset of closed system vaping smoking substitute systems include a device which includes the power source, wherein the device is configured to be physically and electrically coupled to a component including the tank and the heater. In this way, when the tank of a component has been emptied, the device can be reused by connecting it to a new component. Another subset of closed system vaping smoking substitute systems are completely disposable, and intended for one-use only.

There are also "open system" vaping smoking substitute systems which typically have a tank that is configured to be refilled by a user, so the system can be used multiple times.

An example vaping smoking substitute system is the myblu^{™} e-cigarette. The myblu^{™} e cigarette is a closed system which includes a device and a consumable component. The device and consumable component are physically and electrically coupled together by pushing the consumable component into the device. The device includes a rechargeable battery. The consumable component includes a mouthpiece, a sealed tank which contains e-liquid, as well as a vaporiser, which for this system is a heating filament coiled around a portion of a wick which is partially immersed in the e-liquid. The system is activated when a microprocessor on board the device detects a user inhaling through the mouthpiece. When the system is activated, electrical energy is supplied from the power source to the vaporiser, which heats e-liquid from the tank to produce a vapour which is inhaled by a user through the mouthpiece.

Another example vaping smoking substitute system is the blu PRO^{™} e-cigarette. The blu PRO^{™} e cigarette is an open system which includes a device, a (refillable) tank, and a mouthpiece. The device and tank are physically and electrically coupled together by screwing one to the other. The mouthpiece and refillable tank are physically coupled together by screwing one into the other, and detaching the mouthpiece from the refillable tank allows the tank to be refilled with e-liquid. The system is activated by a button on the device. When the system is activated, electrical energy is supplied from the power source to a vaporiser, which heats e-liquid from the tank to produce a vapour which is inhaled by a user through the mouthpiece.

An alternative to the "vaping" approach is the so-called Heated Tobacco ("HT") approach in which tobacco (rather than an e-liquid) is heated or warmed to release vapour. HT is also known as "heat not burn" ("HNB"). The tobacco may be leaf tobacco or reconstituted tobacco. In the HT approach the intention is that the tobacco is heated but not burned, i.e. the tobacco does not undergo combustion.

The heating, as opposed to burning, of the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

A typical HT smoking substitute system may include a device and a consumable component. The consumable component may include the tobacco material. The device and consumable component may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable component (entrained in the airflow) from the location of vaporization to an outlet of the component (e.g. a mouthpiece), the vapour cools and condenses to form an aerosol for inhalation by the user. The aerosol may contain nicotine and/or flavour compounds.

Consumable components are designed exclusively for use with their associated devices and the use of an inappropriate consumable component can lead to reduced user satisfaction. Furthermore, unscrupulous manufacturers may provide lower quality consumable components which may lead not only to an impaired user experience but which may also be hazardous to the user's health. Accordingly, there is a need for an improved aerosol delivery system which addresses at least some of the problems of the known devices and systems.

EP 3378339 A1 discloses an electrically operated aerosol-generating system comprising an aerosol-generating article including at least one component incorporating a taggant within a material of the at least one component, wherein the taggant comprises an identifiable spectroscopic signature.

WO 2019/186147 discloses a control device for an electronic aerosol provision system. The control device is configured to receive a replaceable component to form an electronic aerosol provision system. The control device is further configured to receive an identifier from a replaceable component received by the control device; and to modify one or more characteristics (such as resistance to draw, RTD) of an airflow path through the electronic aerosol provision system dependent upon the received identifier.

### Summary

At its most general, the present invention aims to provide an aerosol delivery system (e.g. a smoking substitute system) that allows for authentication of the consumable component.

According to a first aspect of the present invention, as defined in the present claim 1, there is provided an aerosol delivery system (e.g. a smoking substitute system) comprising:
an aerosol delivery device and an aerosol delivery component engageable with the aerosol delivery device at an interface therebetween, the aerosol delivery component being configured to house an aerosol precursor and the aerosol delivery component comprising a feedback element, wherein:
the aerosol delivery component is associated with validity state information from which it can be determined whether the aerosol delivery component is valid for use with the aerosol delivery device;
the aerosol delivery system is configured to determine from the validity state information whether the aerosol delivery component is valid for use with the aerosol delivery device; and
the feedback element is configured to provide feedback to a user based on the determination of whether the aerosol delivery component is valid for use with the aerosol delivery device.

In this way, the aerosol delivery system is able to provide feedback to the user, directly from the aerosol delivery component itself, about whether or not the aerosol delivery component is valid for use with the aerosol delivery device. Accordingly, a user is informed whether or not the aerosol delivery component is suitable for use with the aerosol delivery device (e.g. authentic). Thus, a user can avoid using counterfeit or inappropriate aerosol delivery components based on the feedback provided by the aerosol delivery component.

Optional features will now be set out. These are applicable singly or in any combination with any aspect.

The validity state information associated with the aerosol delivery component may include an invalidity flag. The invalidity flag may include a "valid" state indicating that the aerosol delivery component is a valid (e.g. authentic) aerosol delivery component, and an "invalid" state indicating that the aerosol delivery component is not valid (e.g. not authentic). The determination of whether the aerosol delivery component is valid for use with the aerosol delivery device may include checking whether the invalidity flag associated with the aerosol delivery component is valid or invalid. If the invalidity flag associated with the aerosol delivery component indicates that the aerosol delivery component is invalid, it may be determined that the aerosol delivery component is not valid for use with the aerosol delivery device.

The validity state information associated with the aerosol delivery component may include compatibility information for determining whether the aerosol delivery component is compatible with the aerosol delivery device. The compatibility information could be provided e.g. in the form of a list of aerosol delivery components which are compatible with the aerosol delivery device. A determination of whether the aerosol delivery component is valid for use with the aerosol delivery device may include checking whether the compatibility information indicates that the aerosol delivery component is compatible or not compatible with the aerosol delivery component.

The validity state information associated with the aerosol delivery component may include an expiry date. A determination of whether the consumable is valid for use with the aerosol delivery device may include checking whether the expiry date has elapsed. If the expiry date is found to have elapsed, it may be determined that the consumable is not valid for use with the aerosol delivery device. The aerosol delivery device may include a clock (for determining a local time and/or date) in order to determine whether an expiry date has elapsed.

The aerosol delivery device may comprise a controller configured to determine from the validity state information whether the aerosol delivery component is valid for use with the aerosol delivery device.

In order for the aerosol delivery device to obtain the validity state information associated with the aerosol delivery component (from which it can be determined whether the aerosol delivery component is valid for use with the aerosol delivery device), the aerosol delivery device may be configured to obtain validity state information associated with the aerosol delivery component by:
reading ID information identifying the aerosol delivery component from an identifier provided with the aerosol delivery component; and
using the ID information to look up the validity state information associated with the aerosol delivery component.

The ID information may uniquely identify the aerosol delivery component, or a batch of aerosol delivery components that includes the aerosol delivery component. The ID information may allow the validity state information associated with the aerosol delivery component to be looked up in a storage device of the aerosol delivery device. In these embodiments, the aerosol delivery device may comprise a storage device, wherein validity state information may be stored in association with ID information identifying the aerosol delivery component at the storage device. The controller of the aerosol delivery device may be configured to look up the validity state information stored in association with the ID information read from the aerosol delivery component in order to obtain the validity state information associated with the aerosol delivery component.

Alternatively, the ID information may allow validity state information associated with the aerosol delivery component to be looked up by an application on a mobile device in communication with the aerosol delivery system, or an application server in communication with the aerosol delivery system/mobile device. In these embodiments, the validity state information may be stored in association with the ID information by the application/mobile device, or at an application server.

In some embodiments, the aerosol delivery component itself may comprise a memory device configured to store the validity state information associated with the aerosol delivery component. The aerosol delivery device may be configured to obtain the validity state information associated with the aerosol delivery component from the memory device in the aerosol delivery component via the interface between the aerosol delivery component and the aerosol delivery device.

The storage device in the aerosol delivery device/memory device in the aerosol delivery component/application on the mobile device/application server may maintain a list of ID information for stolen/lost/out of date aerosol delivery components, and/or a list of aerosol delivery components which are compatible with the aerosol delivery device. Accordingly, the controller can look up the ID information read from the aerosol delivery component in the list stored in the storage device/application/application server, and determine from the validity state information associated with the ID information, whether the aerosol delivery component is valid for use with the aerosol delivery device.

By storing the validity state information in association with ID information at an applications server, the validity state information can be updated by an application server administrator after the aerosol delivery component has left the control of a supplier. This may be useful when the aerosol delivery component is faulty, or has been stolen, and the supplier wants to inhibit its use.

The identifier provided with the aerosol delivery component may be included on or within the aerosol delivery component. For example, the identifier may be (integrally) formed on the aerosol delivery component at a region of the aerosol delivery component configured to interface with the aerosol delivery device, e.g. in the form of an ID number.

The identifier provided with the aerosol delivery component may be provided in the form of a machine readable tag which stores the ID information.

The machine readable tag may be an RFID tag configured to be read by an RFID reader on the aerosol delivery device. The RFID reader and RFID tag may be respectively located so that the RFID reader can read the RFID tag on the aerosol delivery component when (and preferably only when) the aerosol delivery component is engaged with (e.g. physically coupled with) the aerosol delivery device. The machine readable tag may store the ID information in a visual pattern such as a linear or 2D barcode. The machine readable tag may be a QR code readable by a QR reader on the aerosol delivery device.

In some embodiments, the aerosol delivery device may be configured to obtain validity state information associated with the aerosol delivery component (from which it can be determined whether the aerosol delivery component is valid for use with the aerosol delivery device) directly from the aerosol delivery component. For example, the aerosol delivery device may be configured to read the validity state information from a machine readable tag provided with the aerosol delivery component. The machine readable tag may be an RFID tag configured to be readable by an RFID reader on the aerosol delivery device, and the RFID reader and RFID tag may be respectively located in the aerosol delivery device and aerosol delivery component so that the RFID reader can read the RFID tag on the aerosol delivery component when (and preferably only when) the aerosol delivery component is engaged with (e.g. physically coupled with) the aerosol delivery device. The machine readable tag may store the ID information in a visual pattern such as a linear or 2D barcode.

The feedback element of the aerosol delivery component may provide visual feedback, audio feedback and/or haptic feedback.

For example, the feedback element may comprise a visual feedback element, an audio feedback element (e.g. a speaker) and/or a haptic feedback element (e.g. a haptic actuator such as an electric motor and a weight mounted eccentrically on a shaft of the electric motor).

In embodiments in which the feedback element is a visual feedback element, the visual feedback element may comprise one or more light sources e.g. one or more LEDs.

The visual feedback element may comprise an illumination region formed in the aerosol delivery component, and a source of light contained within the aerosol delivery component, wherein the illumination region is configured such that light provided by the light source passes through the illumination region of the aerosol delivery component in order to provide visual feedback to a user based on the determination of whether the aerosol delivery component is valid for use with the aerosol delivery device. In these embodiments, the controller of the aerosol delivery device may be configured to control the light source contained in the aerosol delivery component to illuminate an area of the illumination region based on the determination of whether the aerosol delivery component is valid for use with the aerosol delivery device. In alternative embodiments, a controller in the aerosol delivery component may be configured to control the light source contained in the aerosol delivery component based on the determination of whether the aerosol delivery component is valid for use with the aerosol delivery device.

The light source contained within the aerosol delivery component may be an array of LEDs, for example. The illumination region may be a surface area of the aerosol delivery component.

An intensity of the source of light may be controlled so as to vary the area of illumination. There may be plural sources of light contained within the aerosol delivery component, and a number of source of light may be controlled so as to vary the area of illumination.

The source(s) of light may include a liquid crystal display.

The illumination region of the aerosol delivery component may be made from a diffusing material, such that light passing through the illumination region from the light source(s) is diffused. The illumination region may be formed of polycarbonate or acrylic, for example.

The illumination region may include a plurality of discrete sub-illumination regions. Each sub-illumination may be separated from an adjacent sub-illumination may be separated from an adjacent sub-illumination region by an optically opaque divider.

The feedback element (e.g. visual feedback element, audio feedback element and/or haptic feedback element) may be configured to (under the control of the controller) provide:
a first feedback indication if the aerosol delivery system determines that the aerosol delivery component is valid for use with the aerosol delivery device; and
a second feedback indication if the aerosol delivery system determines that the aerosol delivery component is not valid for use with the aerosol delivery device, the second feedback indication being different from the first feedback indication.

For example, when the feedback element of the aerosol delivery component comprises a visual feedback element (e.g. the illumination region and the light source), the first feedback indication may be the visual feedback element emitting light of a first colour, and the second feedback indication may be the visual feedback element emitting light of a second colour different to the first colour.

For example, the controller may be configured to control the visual feedback element to emit green light when the controller of the aerosol delivery device determines that the aerosol delivery component is valid for use with the aerosol delivery device, and red light when the controller of the aerosol delivery device determines that the aerosol delivery component is not valid for use with the aerosol delivery device.

In this way, the user is taught to use the aerosol delivery component with the aerosol delivery device when green light is emitted, and to not use the aerosol delivery component with the aerosol delivery device when red light is emitted.

The visual feedback may comprise the visual feedback element emitting flashes of light. For example, the first visual feedback may be the visual feedback element emitting flashes of light in a first predetermined sequence, and the second visual feedback may be the visual feedback element emitting flashes of light in a second predetermined sequence, the first and second predetermined sequences being different from one another.

If the feedback element comprises a haptic feedback element, the controller may be configured to control the haptic feedback element (e.g. haptic actuator) to produce a first predetermined haptic sequence when the controller of the aerosol delivery device determines that the aerosol delivery component is valid for use with the aerosol delivery device, and a second predetermined haptic sequence when the controller of the aerosol delivery device determines that the aerosol delivery component is not valid for use with the aerosol delivery device, wherein the first predetermined haptic sequence is different from the second predetermined haptic sequence.

The aerosol delivery system (and in particular the controller of the aerosol delivery device) may be configured to determine whether the aerosol delivery component is valid for use with the aerosol delivery device, and the feedback element is configured to provide feedback to a user based on the determination of whether the aerosol delivery component is valid for use with the aerosol delivery device when (and preferably only when) the aerosol delivery component is engaged with (e.g. physically coupled to) the aerosol delivery device.

The aerosol delivery system may be configured to, if it is determined from the validity state information that the aerosol delivery component is invalid for use with the aerosol delivery device, inhibit use of the aerosol delivery component with the aerosol delivery device. Specifically, the system may be configured to inhibit use of the aerosol delivery component with the aerosol delivery device by disabling the aerosol delivery device such that it does not work with the aerosol delivery component. Disabling the aerosol delivery device such that it does not work with the aerosol delivery component could be achieved, for example, by configuring the aerosol delivery device to not activate (e.g. not pass electrical power to a heating device of the aerosol delivery component) when the aerosol delivery component is engaged with (e.g. physically coupled to) the aerosol delivery device.

The aerosol delivery component may be associated with an aerosol delivery component type information, such as flavour or nicotine strength of the aerosol precursor, for example. The aerosol delivery system may be configured to determine from the aerosol delivery component type information, the type of aerosol delivery component (using similar mechanisms to that described above with regards to validity state information). For example, the aerosol delivery system may be able to determine the flavour of the aerosol precursor housed in the aerosol delivery component by reading ID information identifying the aerosol delivery component from an identifier provided with the aerosol delivery component.

The feedback element may then be configured to provide feedback to a user based on the determination of the aerosol delivery component type (e.g. flavour of the aerosol precursor).

If the feedback element of the aerosol delivery component comprises a visual feedback element, the feedback element may be configured to emit different colours of light based on the determined aerosol delivery component type. For example, if the aerosol delivery system determines that the aerosol delivery component houses a blueberry flavoured aerosol precursor, the feedback element (e.g. illumination region and light source) may emit blue light, whereas if the aerosol delivery system determines that the aerosol delivery component houses lemon flavoured aerosol-precursor, the feedback element may emit yellow light.

The feedback provided to the user based on the determination of whether the aerosol delivery component is valid for use with the aerosol delivery device, and the feedback provided to the user based on the determination of the aerosol delivery component type, may be provided together, or in sequence.

For example, when it is determined that the aerosol delivery component is valid for use with the aerosol delivery device, the feedback element may provide a first feedback indication, wherein the first feedback indication is the visual feedback element emitting a single flash of a first coloured light, and wherein the colour of the coloured light corresponds to the aerosol delivery component type (e.g. flavour). When it is determined that the aerosol delivery component is not valid for use with the aerosol delivery device, the feedback element may provide a second feedback indication (different from the first feedback indication), wherein the second feedback indication is the visual feedback element emitting a double flash of, for example, white light.

Alternatively, the feedback element may first provide the feedback indicating whether the aerosol delivery device is valid for use with the component, and then provide the feedback indicating the aerosol delivery component type.

The aerosol delivery device may house a source of power which may be a battery and/or a capacitor. The aerosol delivery device may comprise a cavity for housing the controller and/or the power source and/or other electrical components. The aerosol delivery device may be an elongate body (i.e. with a greater length then depth/width. It may have a greater width than depth.

The storage device of the aerosol delivery device may be operatively connected to the controller. The storage device may include non-volatile memory. The storage device may include instructions which, when implemented, cause the controller to perform certain tasks or steps of a method.

The aerosol delivery device may comprise a wireless interface, which may be configured to communicate wirelessly with another device, for example a mobile device, e.g. via Bluetooth^{®}. To this end, the wireless interface could include a Bluetooth^{®} antenna. Other wireless communication interfaces, e.g. WiFi^{®}, are also possible. The wireless interface may also be configured to communicate wirelessly with a remote application server.

An airflow (i.e. puff) sensor may be provided that is configured to detect a puff (i.e. inhalation from a user). The airflow sensor may be operatively connected to the controller so as to be able to provide a signal to the controller that is indicative of a puff state (i.e. puffing or not puffing). The airflow sensor may, for example, be in the form of a pressure sensor or an acoustic sensor. The controller may control power supply to a heating element in response to airflow detection by the sensor. The control may be in the form of activation of the heating element in response to a detected airflow. The airflow sensor may form part of the aerosol delivery device. The heating element may be used in a vaporiser to vaporise the aerosol precursor. The vaporiser may be housed in a vaporising chamber.

The aerosol delivery component may be an aerosol-delivery (e.g. a smoking substitute) consumable i.e. in some embodiments the component may be a consumable component for engagement with the aerosol-delivery (e.g. a smoking substitute) device to form the aerosol-delivery (e.g. a smoking substitute) system.

The aerosol delivery device may be configured to receive the consumable component. The device and the consumable component may be configured to be physically coupled together. For example, the consumable component may be at least partially received in a recess of the device, such that there is snap engagement between the device and the consumable component. Alternatively, the device and the consumable component may be physically coupled together by screwing one onto the other, or through a bayonet fitting.

Thus, the consumable component may comprise one or more engagement portions for engaging with the device.

The consumable component may comprise an electrical interface for interfacing with a corresponding electrical interface of the device. One or both of the electrical interfaces may include one or more electrical contacts. Thus, when the device is engaged with the consumable component, the electrical interface may be configured to transfer electrical power from the power source to a heating element of the consumable component. The electrical interface may additionally or alternatively be used to identify when the consumable component is connected to the device.

The aerosol delivery system may comprise an airflow path therethrough, the airflow path extending from an air inlet to an outlet. The outlet may be at a mouthpiece portion of the component. In this respect, a user may draw fluid (e.g. air) into and along the airflow path by inhaling at the outlet (i.e. using the mouthpiece).

The airflow path passes the vaporiser between the air inlet to the air outlet.

The airflow path may comprise a first portion extending from the air inlet towards the vaporiser. The second portion of the airflow path passes through the vaporising chamber to a conduit that extends to the air outlet. The conduit may extend along the axial centre of the component.

References to "downstream" in relation to the airflow path are intended to refer to the direction towards the air outlet/outlet portion. Thus the second and third portions of the airflow path are downstream of the first portion of the airflow path. Conversely, references to "upstream" are intended to refer to the direction towards the air inlet. Thus the first portion of the airflow path (and the air inlet) is upstream of the second/third portions of the airflow path (and the air outlet/outlet portion).

References to "upper", "lower", "above" or "below" are intended to refer to the aerosol delivery component when in an upright/vertical orientation i.e. with elongate (longitudinal/length) axis of the component vertically aligned and with the mouthpiece vertically uppermost.

The aerosol delivery component may comprise a tank for housing the aerosol precursor (e.g. a liquid aerosol precursor). The aerosol precursor may comprise an e-liquid, for example, comprising a base liquid and e.g. nicotine. The base liquid may include propylene glycol and/or vegetable glycerine.

At least a portion of one of the walls defining the tank may be translucent or transparent.

The conduit may extend through the tank with the conduit walls defining an inner region of the tank. In this respect, the tank may surround the conduit e.g. the tank may be annular.

As discussed above, the air flow path passes the vaporiser between the air inlet to the air outlet. The vaporiser may comprise a wick e.g. an elongate wick which may have a cylindrical shape.

The wick may be oriented so as to extend in the direction of the width dimension of the component (perpendicular to the longitudinal axis of the component). Thus the wick may extend in a direction perpendicular to the direction of airflow in the airflow path.

The vaporiser may be disposed in the vaporising chamber. The vaporising chamber may form part of the airflow path.

The wick may comprise a porous material. A portion of the wick may be exposed to airflow in the airflow path. The wick may also comprise one or more portions in contact with liquid aerosol precursor stored in the tank. For example, opposing ends of the wick may protrude into the tank and a central portion (between the ends) may extend across the airflow path so as to be exposed to airflow. Thus, fluid may be drawn (e.g. by capillary action) along the wick, from the tank to the exposed portion of the wick.

The heating element may be in the form of a filament wound about the wick (e.g. the filament may extend helically about the wick). The filament may be wound about the exposed portion of the wick. The heating element is electrically connected (or connectable) to the power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in airflow along the airflow path. This vapour may subsequently cool to form an aerosol e.g. in the conduit.

In a second aspect of the present invention, as defined in the present claim 15, there is provided a method performed by an aerosol delivery system, wherein the aerosol delivery system includes an aerosol delivery device and an aerosol delivery component engageable with the aerosol delivery device at an interface therebetween, the aerosol delivery component being configured to house an aerosol precursor and the aerosol delivery component J comprising a feedback element, and
wherein the aerosol delivery component is associated with validity state information from which it can be determined whether the aerosol delivery component is valid for use with the aerosol delivery device, the method including:
determining from the validity state information whether the aerosol delivery component is valid for use with the aerosol delivery device; and
providing, by the feedback element of the aerosol delivery component, feedback to a user based on the determination of whether the aerosol delivery component is valid for use with the aerosol delivery device.

The steps of the method of the second aspect may only be performed when the aerosol delivery component is engaged with (e.g. physically coupled with) the aerosol delivery device.

The method may also comprise the step of, if it is determined from the validity state information that the aerosol delivery component is invalid for use with the aerosol delivery device, inhibit use of the aerosol delivery device.

The method of the second aspect may be performed by the aerosol delivery system of the first aspect.

In a third aspect there is provided a method of using the aerosol-delivery (e.g. smoking substitute) system according to the first aspect, the method comprising engaging the aerosol delivery component with the aerosol-delivery (e.g. smoking substitute) device (as described above) having a power source so as to electrically connect the power source to the aerosol delivery component (i.e. to the vaporiser of the consumable component).

The disclosure includes the combination of the aspects and preferred features described except where such a combination is clearly impermissible or expressly avoided.

### BRIEF DESCRIPTION OF THE DRAWINGS

So that further aspects and features thereof may be appreciated, embodiments will now be discussed in further detail with reference to the accompanying figures, in which:
- Fig. 1A is a front schematic view of a smoking substitute system;
- Fig. 1B is a front schematic view of a device of the system;
- Fig. 1C is a front schematic view of a component of the system;
- Fig. 2A is a schematic of the components of the device;
- Fig. 2B is a schematic of the components of the component; and
- Fig. 3 is a section view of the component.

### DETAILED DESCRIPTION OF THE EMBODIMENTS

Aspects and embodiments will now be discussed with reference to the accompanying figures. Further aspects and embodiments will be apparent to those skilled in the art.

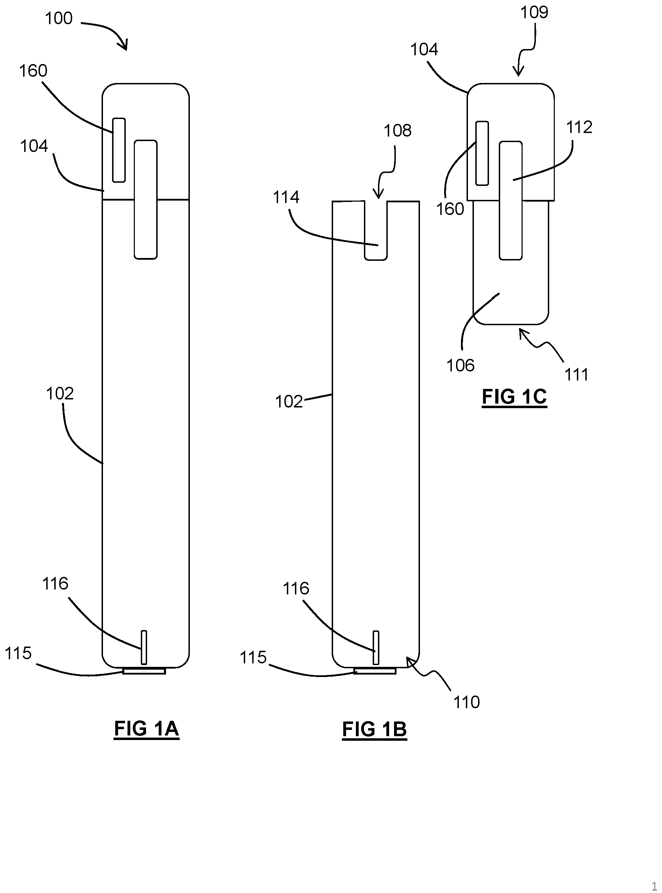

Fig. 1A shows a first embodiment of a smoking substitute system 100. In this example, the smoking substitute system 100 includes a device 102 and a component 104. The component 104 may alternatively be referred to as a "pod", "cartridge" or "cartomizer". In other examples, a tank of the component 104 may be accessible for refilling the device.

In this example, the smoking substitute system 100 is a closed system vaping system, wherein the component 104 includes a sealed tank 106 and is intended for single-use only. The component 104 is removably engageable with the device 102 (i.e. for removal and replacement). Fig. 1A shows the smoking substitute system 100 with the device 102 physically coupled to the component 104, Fig. 1B shows the device 102 of the smoking substitute system 100 without the component 104, and Fig. 1C shows the component 104 of the smoking substitute system 100 without the device 102.

The device 102 and the component 104 are configured to be physically coupled together by pushing the component 104 into a cavity at an upper end 108 of the device 102, such that there is an interference fit between the device 102 and the component 104. In other examples, the device 102 and the component may be coupled by screwing one onto the other, or through a bayonet fitting.

The component 104 includes a mouthpiece (not shown in Fig. 1A, 1B or 1C) at an upper end 109 of the component 104, and one or more air inlets (not shown) in fluid communication with the mouthpiece such that air can be drawn into and through the component 104 when a user inhales through the mouthpiece. The tank 106 containing e-liquid is located at the lower end 111 of the component 104.

The component 104 includes a visual feedback element 160. The visual feedback element 160 provides visual feedback to a user of the system 100 about whether or not the component 104 is valid, and therefore authorized, for use with the device 102, when the device 102 and component 104 are physically coupled together.

The tank 106 includes a window 112, which allows the amount of e-liquid in the tank 106 to be visually assessed. The device 102 includes a slot 114 so that the window 112 of the component 104 can be seen whilst the rest of the tank 106 is obscured from view when the component 104 is inserted into the cavity at the upper end 108 of the device 102.

The lower end 110 of the device 102 also includes a light 116 (e.g. an LED) located behind a small translucent cover. The light 116 may be configured to illuminate when the smoking substitute system 100 is activated. Whilst not shown, the component 104 may identify itself to the device 102, via an electrical interface, RFID chip, or barcode.

The lower end 110 of the device 102 also includes a charging connection 115, which is usable to charge a battery within the device 102. The charging connection 115 can also be used to transfer data to and from the device, for example to update firmware thereon.

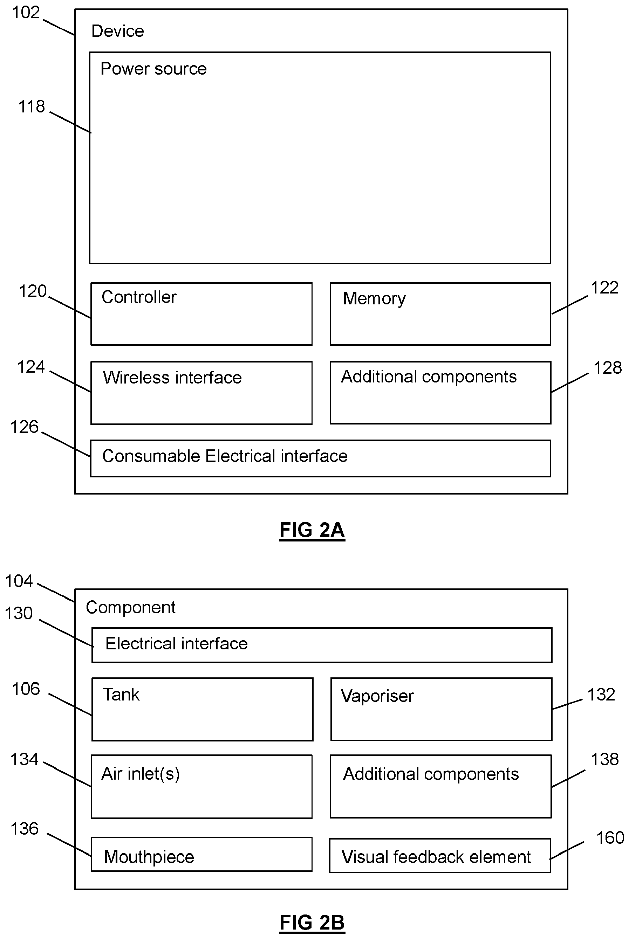

Figs. 2A and 2B are schematic drawings of the device 102 and component 104. As is apparent from Fig. 2A, the device 102 includes a power source 118, a controller 120, a memory 122, a wireless interface 124, an electrical interface 126, and, optionally, one or more additional components 128.

The power source 118 is preferably a battery, more preferably a rechargeable battery. The controller 120 may include a microprocessor, for example. The memory 122 preferably includes non-volatile memory. The memory may include instructions which, when implemented, cause the controller 120 to perform certain tasks or steps of a method.

The wireless interface 124 is preferably configured to communicate wirelessly with another device, for example a mobile device, e.g. via Bluetooth^{®}. To this end, the wireless interface 124 could include a Bluetooth^{®} antenna. Other wireless communication interfaces, e.g. WiFi^{®}, are also possible. The wireless interface 124 may also be configured to communicate wirelessly with a remote server.

The electrical interface 126 of the device 102 may include one or more electrical contacts. The electrical interface 126 may be located in a base of the aperture in the upper end 108 of the device 102. When the device 102 is physically coupled to the component 104, the electrical interface 126 is configured to transfer electrical power from the power source 118 to the component 104 (i.e. upon activation of the smoking substitute system 100).

The electrical interface 126 may also be used to identify and/or authenticate the component 104 for use with the device. For example, the component 104 may be a particular flavour and/or have a certain concentration of nicotine (which may be identified by the electrical interface 126). The component 104 may be associated with validity state information from which it can be determined whether the component 104 is valid for use with the device 102. This can be indicated to the controller 120 of the device 102 when the component 104 is connected to the device 102. Additionally, or alternatively, there may be a separate communication interface provided in the device 102 and a corresponding communication interface in the component 104 such that, when connected, the component 104 can identify itself to the device 102.

The additional components 128 of the device 102 may comprise the light 116 discussed above.

The additional components 128 of the device 102 also comprises the charging connection 115 configured to receive power from the charging station (i.e. when the power source 118 is a rechargeable battery). This may be located at the lower end 110 of the device 102.

The additional components 128 of the device 102 may, if the power source 118 is a rechargeable battery, include a battery charging control circuit, for controlling the charging of the rechargeable battery. However, a battery charging control circuit could equally be located in the charging station (if present).

The additional components 128 of the device 102 may include a sensor, such as an airflow (i.e. puff) sensor for detecting airflow in the smoking substitute system 100, e.g. caused by a user inhaling through a mouthpiece 136 of the component 104. The smoking substitute system 100 may be configured to be activated when airflow is detected by the airflow sensor. This sensor could alternatively be included in the component 104. The airflow sensor can be used to determine, for example, how heavily a user draws on the mouthpiece or how many times a user draws on the mouthpiece in a particular time period.

The additional components 128 of the device 102 may include a user input, e.g. a button. The smoking substitute system 100 may be configured to be activated when a user interacts with the user input (e.g. presses the button). This provides an alternative to the airflow sensor as a mechanism for activating the smoking substitute system 100.

As shown in Fig. 2B, the component 104 includes the tank 106, an electrical interface 130, a vaporiser 132, one or more air inlets 134, a mouthpiece 136, and one or more additional components 138.

The electrical interface 130 of the component 104 may include one or more electrical contacts. The electrical interface 126 of the device 102 and an electrical interface 130 of the component 104 are configured to contact each other and thereby electrically couple the device 102 to the component 104 when the lower end 111 of the component 104 is inserted into the upper end 108 of the device 102 (as shown in Fig. 1A). In this way, electrical energy (e.g. in the form of an electrical current) is able to be supplied from the power source 118 in the device 102 to the vaporiser 132 in the component 104.

The vaporiser 132 is configured to heat and vaporise e-liquid contained in the tank 106 using electrical energy supplied from the power source 118. As will be described further below, the vaporiser 132 includes a heating filament and a wick. The wick draws e-liquid from the tank 106 and the heating filament heats the e-liquid to vaporise the e-liquid.

The one or more air inlets 134 are preferably configured to allow air to be drawn into the smoking substitute system 100, when a user inhales through the mouthpiece 136. When the component 104 is physically coupled to the device 102, the air inlets 134 receive air, which flows to the air inlets 134 along a gap between the device 102 and the lower end 111 of the component 104.

In operation, a user activates the smoking substitute system 100, e.g. through interaction with a user input forming part of the device 102 or by inhaling through the mouthpiece 136 as described above. Upon activation, the controller 120 may supply electrical energy from the power source 118 to the vaporiser 132 (via electrical interfaces 126, 130), which may cause the vaporiser 132 to heat e-liquid drawn from the tank 106 to produce a vapour which is inhaled by a user through the mouthpiece 136.

An example of one of the one or more additional components 138 of the component 104 is an interface for obtaining an identifier of the component 104. This interface may be, for example, an RFID reader, a barcode, a QR code reader, or an electronic interface which is able to identify the component for use in an authentication method. The component 104 may therefore include any one or more of an RFID chip, a barcode or QR code, or memory within which is an identifier and which can be interrogated via the electronic interface in the device 102.

In some examples, the controller 120 of the device 102 may be able to obtain validity state information associated with the consumable 104 directly from the RFID chip, barcode or QR code on the component 104.

The additional components 138 of the component 104 may comprise a memory (not shown). In some examples, the memory may store validity state information associated with the component 104. This validity state information may be communicated to the controller 120 of the device 102 via the electrical interfaces 126, 130, for example, in order for the controller 120 to obtain the validity state information from the component 104.

In other examples, ID information identifying the component may be read by the device 102 from the RFID chip, barcode or QR code of the consumable 104, and the controller 120 may use the ID information to look up validity state information associated with the component in the memory 122.

The controller 120 can then determine from the validity state information associated with the component 104 whether the component 104 is valid for use with the device 102.

Feedback based on the determination of whether the component 104 is valid for use with the device 102 can then be communicated to a user via the visual feedback element 160 provided in the component 104. In some examples, the visual feedback element 160 comprises an illumination region formed in the component 104 (e.g. a surface area of the component 104), and a light source contained within the component, wherein the illumination region is configured such that light provided by the light source passes through the illumination region of the component in order to provide visual feedback to a user based on the determination of whether the component 104 is valid for use with device 102. The illumination region of the visual feedback element may comprise a translucent cover, and the light source may be located behind the translucent cover. The light source may be, in this example, one or more LEDs. The light source may be a group of LEDs operable in a combination of colours. The illumination region may be formed of a diffusing material, for example polycarbonate, such that light emitted from the LEDs is diffused as it is transmitted through the illumination region.

Additionally, or alternatively, the illumination region of the visual feedback element 160 may have a thickness which is thinner than the remaining surface of the component. Further additionally, or alternatively, a liquid crystal display (LCD) may be provided in the illumination region and may be behind the diffusion material (or alternatively may be present as an outermost surface of the component).

In other examples, the visual feedback element may comprise a single light source, such as a single LED. In further examples, the component 104 may comprise an audio feedback element (e.g. a speaker) and/or a haptic feedback element (e.g. a haptic actuator) instead of, or in addition to, the visual feedback element 160.

The controller 120 may be configured to control the visual feedback element 160 to emit different colours of light depending on whether or not the component 104 is determined to be valid for use with the device 102. For example, if the component 104 is determined as being valid for use with the device 102, the visual feedback element 160 may emit green light, whereas if the component 104 is determined as being invalid (and therefore not authenticated) for use with the device 102, the visual feedback element 160 may emit red light.

Where it is determined that the component 104 is not valid for use with the device 102, the controller 120 may prevent the supply of power from the power source 118 to the vaporiser 132 via the electrical interfaces 126, 130.

It should be appreciated that the smoking substitute system 100 shown in figures 1A to 2B is just one exemplary implementation of a smoking substitute system.

The controller 120 may also be configured to determine a flavour and/or nicotine strength of the aerosol precursor stored in tank 106 from the RFID chip, barcode, or QR code on the component 104.

The controller 120 may then be able to control the visual feedback element 160 to emit different colours of light depending on the determined flavour and/or nicotine strength. For example, if the aerosol precursor in component 104 is determined to be blueberry flavoured aerosol-precursor, the visual feedback element 160 may emit blue light, whereas if the aerosol precursor in the component 104 is determined to be lemon flavoured, the visual feedback element 160 may emit yellow light.

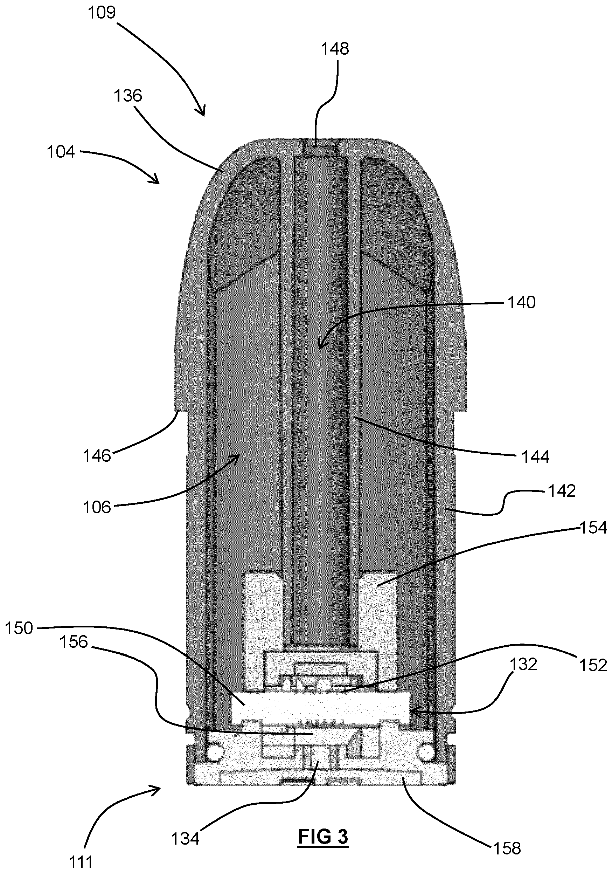

Fig. 3 is a section view of the component 104 described above. The component 104 comprises a tank 106 for storing e-liquid, a mouthpiece 136 and a conduit 140 extending along a longitudinal axis of the component 104. In the illustrated embodiment the conduit 140 is in the form of a tube having a substantially circular transverse cross-section (i.e. transverse to the longitudinal axis). The tank 106 surrounds the conduit 140, such that the conduit 140 extends centrally through the tank 106.

A tank housing 142 of the tank 106 defines an outer casing of the component 104, whilst a conduit wall 144 defines the conduit 140. The tank housing 142 extends from the lower end 111 of the component 104 to the mouthpiece 136 at the upper end 109 of the component 104. At the junction between the mouthpiece 136 and the tank housing 142, the mouthpiece 136 is wider than the tank housing 142, so as to define a lip 146 that overhangs the tank housing 142. This lip 146 acts as a stop feature when the component 104 is inserted into the device 102 (i.e. by contact with an upper edge of the device 102).

The tank 106, the conduit 140 and the mouthpiece 136 are integrally formed with each other so as to form a single unitary component and may e.g. be formed by way of an injection moulding process. Such a component may be formed of a thermoplastic material such as polypropylene.

The mouthpiece 136 comprises a mouthpiece aperture 148 defining an outlet of the conduit 140. The vaporiser 132 is fluidly connected to the mouthpiece aperture 148 and is located in a vaporising chamber 156 of the component 104. The vaporising chamber 156 is downstream of the inlet 134 of the component 104 and is fluidly connected to the mouthpiece aperture 148 (i.e. outlet) by the conduit 140.

The vaporiser 132 comprises a porous wick 150 and a heater filament 152 coiled around the porous wick 150. The wick 150 extends transversely across the chamber vaporising 156 between sidewalls of the chamber 156 which form part of an inner sleeve 154 of an insert 158 that defines the lower end 111 of the component 104 that connects with the device 102. The insert 158 is inserted into an open lower end of the tank 106 so as to seal against the tank housing 142.

In this way, the inner sleeve 154 projects into the tank 106 and seals with the conduit 140 (around the conduit wall 144) so as to separate the vaporising chamber 156 from the e-liquid in the tank 106. Ends of the wick 150 project through apertures in the inner sleeve 154 and into the tank 106 so as to be in contact with the e-liquid in the tank 106. In this way, e-liquid is transported along the wick 150 (e.g. by capillary action) to a central portion of the wick 150 that is exposed to airflow through the vaporising chamber 156. The transported e-liquid is heated by the heater filament 152 (when activated e.g. by detection of inhalation), which causes the e-liquid to be vaporised and to be entrained in air flowing past the wick 150. This vaporised liquid may cool to form an aerosol in the conduit 140, which may then be inhaled by a user.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances.

## Claims

1. An aerosol delivery system (100) comprising:
an aerosol delivery device (102) and an aerosol delivery component (104) engageable with the aerosol delivery device (102) at an interface therebetween, the aerosol delivery component (104) being configured to house an aerosol precursor, and the aerosol delivery component (104) comprising a feedback element (160), wherein:
the aerosol delivery component (104) is associated with validity state information from which it can be determined whether the aerosol delivery component (104) is valid for use with the aerosol delivery device (102);
the aerosol delivery system (100) is configured to determine from the validity state information whether the aerosol delivery component (104) is valid for use with the aerosol delivery device (102); and
the feedback element (160) is configured to provide feedback to a user based on the determination of whether the aerosol delivery component (104) is valid for use with the aerosol delivery device (102).

2. The aerosol delivery system (100) of claim 1, wherein the aerosol delivery device (102) comprises a controller (120), the controller being configured to determine from the validity state information whether the aerosol delivery component (104) is valid for use with the aerosol delivery device (102).

3. The aerosol delivery system (100) of claim 2, wherein the aerosol delivery device (102) is configured to obtain validity state information associated with the aerosol delivery component (104) by:
reading ID information identifying the aerosol delivery component (104) from an identifier provided with the aerosol delivery component (104); and
using the ID information to look up the validity state information associated with the aerosol deliverycomponent (104).

4. The aerosol delivery system (100) of claim 3, wherein:
the aerosol delivery system (100) comprises a storage device (122), the storage device (122) being configured to store validity state information in association with ID information identifying the aerosol delivery component (104); and
the controller (120) of the aerosol delivery device (102) is configured to look up the validity state information stored in association with the ID information read from the aerosol delivery component (104) in order to obtain the validity state information associated with the aerosol delivery component (104).

5. The aerosol delivery system (100) of claim 3 or claim 4, wherein the identifier provided with the aerosol delivery component (104) is formed on the aerosol delivery component (104) and is provided in the form of a machine readable tag which stores the ID information.

6. The aerosol delivery system (100) of claim 2, wherein the aerosol delivery device (102) is configured to obtain validity state information associated with the aerosol delivery component (104) directly from the aerosol delivery component (104) by reading validity state information from a machine readable tag provided with the aerosol delivery component (104).

7. The aerosol delivery system (100) of claim 5 or claim 6, wherein the machine readable tag is an RFID tag configured to be read by an RFID reader on the aerosol delivery device (102), and wherein the RFID reader and RFID tag are respectively located so that the RFID reader can read the RFID tag on the aerosol delivery component (104) when the aerosol delivery component (104) is engaged with the aerosol delivery device (102).

8. The aerosol delivery system (100) of any preceding claim, wherein the feedback element (160) of the aerosol delivery component (104) comprises a visual feedback element (160).

9. The aerosol delivery system (100) of claim 8, wherein the visual feedback element (160) comprises an illumination region formed in the aerosol delivery component (104), and a light source contained within the aerosol delivery component (104), wherein the illumination region is configured such that light provided by the light source passes through the illumination region of the aerosol delivery component (104) in order to provide visual feedback to a user based on the determination of whether the aerosol delivery component (104) is valid for use with the aerosol delivery device (102).

10. The aerosol delivery system (100) of any preceding claim, wherein the feedback element (160) is configured to provide:
a first feedback indication if the aerosol delivery system (100) determines that the aerosol delivery component (104) is valid for use with the aerosol delivery device (102); and
a second feedback indication if the aerosol delivery system (100) determines that the aerosol delivery component (104) is not valid for use with the aerosol delivery device (102), the second feedback indication being different from the first feedback indication.

11. The aerosol delivery system (100) of claim 10, wherein the feedback element (160) of the aerosol delivery component (104) comprises a visual feedback element (160), the first feedback indication is the visual feedback element (160) emitting light of a first colour, and the second feedback indication is the visual feedback element (160) emitting light of a second colour, the second colour being different to the first colour.

12. The aerosol delivery system (100) of any preceding claim, wherein the aerosol delivery system (100) is configured to determine whether the aerosol delivery component (104) is valid for use with the aerosol delivery device (102), and the feedback element (160) is configured to provide feedback to the user based on the determination of whether the aerosol delivery component (104) is valid for use with the aerosol delivery device (102), when the aerosol delivery component (104) is engaged with the aerosol delivery device (102).

13. The aerosol delivery system (100) of any preceding claim, wherein the aerosol delivery system (100) is configured to, if it is determined from the validity state information that the aerosol delivery component (104) is invalid for use with the aerosol delivery device (102), inhibit use of the aerosol delivery component (104) with the aerosol delivery device (102).

14. The aerosol delivery system (100) of any preceding claim, wherein the aerosol delivery component (104) comprises a memory device configured to store the validity state information associated with the aerosol delivery component (104), and wherein the aerosol delivery device (102) is configured to obtain the validity state information associated with the aerosol delivery component (104) and stored in the memory device of the aerosol delivery component (104) via the interface between the aerosol delivery component (104) and the aerosol delivery device (102).

15. A method performed by an aerosol delivery system (100), wherein the aerosol delivery system includes an aerosol delivery device (102) and an aerosol delivery component (104) engageable with the aerosol delivery device (102) at an interface therebetween, the aerosol delivery component (104) being configured to house an aerosol precursor, and the aerosol delivery component (104) comprising a feedback element (160), and wherein the aerosol delivery component (104) is associated with validity state information from which it can be determined whether the aerosol delivery component (104) is valid for use with the aerosol delivery device (102), the method including:
determining from the validity state information whether the aerosol delivery component (104) is valid for use with the aerosol delivery device (102); and
providing, by the feedback element of the aerosol delivery component (104), feedback to a user based on the determination of whether the aerosol delivery component (104) is valid for use with the aerosol delivery device (102).

## Patentansprüche

1. Aerosolabgabesystem (100), das Folgendes umfasst:
eine Aerosolabgabevorrichtung (102) und eine Aerosolabgabekomponente (104), die mit der Aerosolabgabevorrichtung (102) an einer Grenzfläche zwischen diesen in Eingriff bringbar ist, wobei die Aerosolabgabekomponente (104) ausgelegt ist, um einen Aerosolvorläufer aufzunehmen, und die Aerosolabgabekomponente (104) ein Rückmeldungselement (160) umfasst; wobei
die Aerosolabgabekomponente (104) Gültigkeitszustandsinformationen zugeordnet ist, anhand derer bestimmt werden kann, ob die Aerosolabgabekomponente (104) für die Verwendung mit der Aerosolabgabevorrichtung (102) gültig ist;
wobei das Aerosolabgabesystem (100) ausgelegt ist, um anhand der Gültigkeitszustandsinformationen zu bestimmen, ob die Aerosolabgabekomponente (104) für die Verwendung mit der Aerosolabgabevorrichtung (102) gültig ist; und
das Rückmeldungselement (160) ausgelegt ist, um für einen Benutzer eine Rückmeldung basierend auf der Bestimmung, ob die Aerosolabgabekomponente (104) für die Verwendung mit der Aerosolabgabevorrichtung (102) gültig ist, bereitzustellen.

2. Aerosolabgabesystem (100) nach Anspruch 1, wobei die Aerosolabgabevorrichtung (102) eine Steuerung (120) umfasst, wobei die Steuerung ausgelegt ist, um anhand der Gültigkeitszustandsinformationen zu bestimmen, ob die Aerosolabgabekomponente (104) für die Verwendung mit der Aerosolabgabevorrichtung (102) gültig ist.

3. Aerosolabgabesystem (100) nach Anspruch 2, wobei die Aerosolabgabevorrichtung (102) ausgelegt ist, um der Aerosolabgabekomponente (104) zugeordnete Gültigkeitszustandsinformationen zu erhalten, indem sie:
ID-Informationen, die die Aerosolabgabekomponente (104) identifizieren, aus einem mit der Aerosolabgabekomponente (104) bereitgestellten Identifikator ausliest; und
die ID-Informationen verwendet, um die der Aerosolabgabekomponente (104) zugeordneten Gültigkeitszustandsinformationen nachzuschlagen.

4. Aerosolabgabesystem (100) nach Anspruch 3, wobei
das Aerosolabgabesystem (100) eine Speichervorrichtung (122) umfasst, wobei die Speichervorrichtung (122) ausgelegt ist, um Gültigkeitszustandsinformationen zugeordnet zu ID-Informationen, die die Aerosolabgabekomponente (104) identifizieren, zu speichern; und
die Steuerung (120) der Aerosolabgabevorrichtung (102) ausgelegt ist, um die Gültigkeitszustandsinformationen, die zugeordnet zu den aus der Aerosolabgabekomponente (104) ausgelesenen ID-Informationen gespeichert sind, nachzuschlagen, um die der Aerosolabgabekomponente (104) zugeordneten Gültigkeitszustandsinformationen zu erhalten.

5. Aerosolabgabesystem (100) nach Anspruch 3 oder 4, wobei der mit der Aerosolabgabekomponente (104) bereitgestellte Identifikator auf der Aerosolabgabekomponente (104) ausgebildet ist und in Form einer maschinenlesbaren Markierung, die die ID-Informationen speichert, bereitgestellt ist.

6. Aerosolabgabesystem (100) nach Anspruch 2, wobei die Aerosolabgabevorrichtung (102) ausgelegt ist, um der Aerosolabgabekomponente (104) zugeordnete Gültigkeitszustandsinformationen direkt von der Aerosolabgabekomponente (104) zu erhalten, indem sie Gültigkeitszustandsinformationen aus einer mit der Aerosolabgabekomponente (104) zugeordneten computerlesbaren Markierung ausliest.

7. Aerosolabgabesystem (100) nach Anspruch 5 oder 6, wobei die maschinenlesbare Markierung eine RFID-Markierung ist, die ausgelegt ist, auf einem RFID-Lesegerät auf der Aerosolabgabevorrichtung (102) ausgelesen zu werden, und wobei das RFID-Lesegerät und die RFID-Markierung jeweils so angeordnet sind, dass das RFID-Lesegerät die RFID-Markierung auf der Aerosolabgabekomponente (104) auslesen kann, wenn die Aerosolabgabekomponente (104) mit der Aerosolabgabevorrichtung (102) in Eingriff steht.

8. Aerosolabgabesystem (100) nach einem der vorangegangenen Ansprüche, wobei das Rückmeldungselement (160) der Aerosolabgabekomponente (104) ein visuelles Rückmeldungselement (160) umfasst.

9. Aerosolabgabesystem (100) nach Anspruch 8, wobei das visuelle Rückmeldungselement (160) eine in der Aerosolabgabekomponente (104) ausgebildete Leuchtregion und eine in der Aerosolabgabekomponente (104) enthaltene Lichtquelle umfasst, wobei die Leuchtregion so ausgelegt ist, dass durch die Lichtquelle bereitgestelltes Licht durch die Leuchtregion der Aerosolabgabekomponente (104) tritt, um für einen Benutzer eine visuelle Rückmeldung basierend auf der Bestimmung, ob die Aerosolabgabekomponente (104) für die Verwendung mit der Aerosolabgabevorrichtung (102) gültig ist, bereitzustellen.

10. Aerosolabgabesystem (100) nach einem der vorangegangenen Ansprüche, wobei das Rückmeldungselement (160) ausgelegt ist, um Folgendes bereitzustellen:
eine erste Rückmeldungsangabe, wenn das Aerosolabgabesystem (100) bestimmt, dass die Aerosolabgabekomponente (104) für die Verwendung mit der Aerosolabgabevorrichtung (102) gültig ist,
eine zweite Rückmeldungsangabe, wenn das Aerosolabgabesystem (100) bestimmt, dass die Aerosolabgabekomponente (104) nicht für die Verwendung mit der Aerosolabgabevorrichtung (102) gültig ist, wobei sich die zweite Rückmeldungsangabe von der ersten Rückmeldungsangabe unterscheidet.

11. Aerosolabgabesystem (100) nach Anspruch 10, wobei das Rückmeldungselement (160) der Aerosolabgabekomponente (104) ein visuelles Rückmeldungselement (160) umfasst, die erste Rückmeldungsangabe darin besteht, dass das visuelle Rückmeldungselement (160) Licht einer ersten Farbe emittiert, und die zweite Rückmeldungsangabe darin besteht, dass das visuelle Rückmeldungselement (160) Licht einer zweiten Farbe emittiert, wobei sich das zweite Licht vom ersten Licht unterscheidet.

12. Aerosolabgabesystem (100) nach einem vorangegangenen Anspruch, wobei das Aerosolabgabesystem (100) ausgelegt ist, um zu bestimmen, ob Aerosolabgabekomponente (104) für die Verwendung mit der Aerosolabgabevorrichtung (102) gültig ist, und das Rückmeldungselement (160) ausgelegt ist, um für einen Benutzer eine Rückmeldung basierend auf der Bestimmung, ob die Aerosolabgabekomponente (104) für die Verwendung mit der Aerosolabgabevorrichtung (102) gültig ist, bereitzustellen, wenn die Aerosolabgabekomponente (104) mit der Aerosolabgabevorrichtung (102) in Eingriff steht.

13. Aerosolabgabesystem (100) nach einem vorangegangenen Anspruch, wobei das Aerosolabgabesystem (100) ausgelegt ist, um, wenn anhand der Gültigkeitszustandsinformationen bestimmt wird, dass die Aerosolabgabekomponente (104) nicht zur Verwendung mit der Aerosolabgabevorrichtung (102) gültig ist, die Verwendung der Aerosolabgabekomponente (104) mit der Aerosolabgabevorrichtung (102) zu hemmen.

14. Aerosolabgabesystem (100) nach einem vorangegangenen Anspruch, wobei die Aerosolabgabekomponente (104) eine Speichervorrichtung umfasst, die ausgelegt ist, um die der Aerosolabgabekomponente (104) zugeordneten Gültigkeitszustandsinformationen zu speichern, und wobei die Aerosolabgabevorrichtung (102) ausgelegt ist, um die der Aerosolabgabekomponente (104) zugeordneten und in der Speichervorrichtung der Aerosolabgabekomponente (104) gespeicherten Gültigkeitszustandsinformationen über die Grenzfläche zwischen der Aerosolabgabekomponente (104) und der Aerosolabgabevorrichtung (102) zu erhalten.

15. Verfahren, das durch ein Aerosolabgabesystem (100) durchgeführt wird, wobei das Aerosolabgabesystem Folgendes umfasst:
eine Aerosolabgabevorrichtung (102) und eine Aerosolabgabekomponente (104), die mit der Aerosolabgabevorrichtung (102) an einer Grenzfläche zwischen diesen in Eingriff bringbar ist, wobei die Aerosolabgabekomponente (104) ausgelegt ist, um einen Aerosolvorläufer aufzunehmen, und die Aerosolabgabekomponente (104) ein Rückmeldungselement (160) umfasst, und wobei die Aerosolabgabekomponente (104) Gültigkeitszustandsinformationen zugeordnet ist, anhand derer bestimmt werden kann, ob die Aerosolabgabekomponente (104) für die Verwendung mit der Aerosolabgabevorrichtung (102) gültig ist;
wobei das Verfahren Folgendes umfasst:
Bestimmen, ob die Aerosolabgabekomponente (104) für die Verwendung mit der Aerosolabgabevorrichtung (102) gültig ist, anhand der Gültigkeitszustandsinformationen; und
Bereitstellen einer Rückmeldung für den Benutzer durch das Rückmeldungselement der Aerosolabgabekomponente (104) basierend auf der Bestimmung, ob die Aerosolabgabekomponente (104) für die Verwendung mit der Aerosolabgabevorrichtung (102) gültig ist.

## Revendications

1. Système de distribution d'aérosol (100) comprenant :
un dispositif de distribution d'aérosol (102) et un composant de distribution d'aérosol (104) pouvant être mis en prise avec le dispositif de distribution d'aérosol (102) au niveau d'une interface entre eux, le composant de distribution d'aérosol (104) étant configuré pour loger un précurseur d'aérosol, et le composant de distribution d'aérosol (104) comprenant un élément de rétroaction (160), dans lequel :
le composant de distribution d'aérosol (104) est associé à des informations d'état de validité à partir desquelles il est possible de déterminer si le composant de distribution d'aérosol (104) est valide pour une utilisation avec le dispositif de distribution d'aérosol (102) ;
le système de distribution d'aérosol (100) est configuré pour déterminer à partir des informations d'état de validité si le composant de distribution d'aérosol (104) est valide pour une utilisation avec le dispositif de distribution d'aérosol (102) ; et
l'élément de rétroaction (160) est configuré pour fournir une rétroaction à un utilisateur sur la base de la détermination de la validité du composant de distribution d'aérosol (104) pour une utilisation avec le dispositif de distribution d'aérosol (102).

2. Système de distribution d'aérosol (100) selon la revendication 1, dans lequel le dispositif de distribution d'aérosol (102) comprend un dispositif de commande (120), le dispositif de commande étant configuré pour déterminer à partir des informations d'état de validité si le composant de distribution d'aérosol (104) est valide pour une utilisation avec le dispositif de distribution d'aérosol (102).

3. Système de distribution d'aérosol (100) selon la revendication 2, dans lequel le dispositif de distribution d'aérosol (102) est configuré pour obtenir des informations d'état de validité associées au composant de distribution d'aérosol (104) en : lisant des informations d'identification identifiant le composant de distribution d'aérosol (104) à partir d'un identifiant fourni avec le composant de distribution d'aérosol (104) ; et
utilisant les informations d'identification pour rechercher les informations d'état de validité associées au composant de distribution d'aérosol (104).

4. Système de distribution d'aérosol (100) selon la revendication 3, dans lequel :
le système de distribution d'aérosol (100) comprend un dispositif de stockage (122), le dispositif de stockage (122) étant configuré pour stocker des informations d'état de validité en association avec des informations d'identification identifiant le composant de distribution d'aérosol (104) ; et
le dispositif de commande (120) du dispositif de distribution d'aérosol (102) est configuré pour rechercher les informations d'état de validité
stockées en association avec les informations d'identification lues à partir du composant de distribution d'aérosol (104) afin d'obtenir les informations d'état de validité associées au composant de distribution d'aérosol (104).

5. Système de distribution d'aérosol (100) selon la revendication 3 ou la revendication 4, dans lequel l'identifiant fourni avec le composant de distribution d'aérosol (104) est formé sur le composant de distribution d'aérosol (104) et est fourni sous la forme d'une étiquette lisible par machine qui stocke les informations d'identification.

6. Système de distribution d'aérosol (100) selon la revendication 2, dans lequel le dispositif de distribution d'aérosol (102) est configuré pour obtenir des informations d'état de validité associées au composant de distribution d'aérosol (104) directement à partir du composant de distribution d'aérosol (104) en lisant des informations d'état de validité à partir d'une étiquette lisible par machine fournie avec le composant de distribution d'aérosol (104).

7. Système de distribution d'aérosol (100) selon la revendication 5 ou la revendication 6, dans lequel l'étiquette lisible par machine est une étiquette RFID configurée pour être lue par un lecteur RFID sur le dispositif de distribution d'aérosol (102), et dans lequel le lecteur RFID et l'étiquette RFID sont positionnés respectivement de sorte que le lecteur RFID peut lire l'étiquette RFID sur le composant de distribution d'aérosol (104) lorsque le composant de distribution d'aérosol (104) est mis en prise avec le dispositif de distribution d'aérosol (102).

8. Système de distribution d'aérosol (100) selon l'une quelconque des revendications précédentes, dans lequel l'élément de rétroaction (160) du composant de distribution d'aérosol (104) comprend un élément de rétroaction visuelle (160).

9. Système de distribution d'aérosol (100) selon la revendication 8, dans lequel l'élément de rétroaction visuelle (160) comprend une région d'éclairage formée dans le composant de distribution d'aérosol (104), et une source de lumière contenue dans le composant de distribution d'aérosol (104),
dans lequel la région d'éclairage est configurée de telle sorte que de la lumière fournie par la source de lumière passe à travers la région d'éclairage du composant de distribution d'aérosol (104) afin de fournir une rétroaction visuelle à un utilisateur sur la base de la détermination de la validité ou non du composant de distribution d'aérosol (104) pour une utilisation avec le dispositif de distribution d'aérosol (102).

10. Système de distribution d'aérosol (100) selon l'une quelconque des revendications précédentes, dans lequel l'élément de rétroaction (160) est configuré pour fournir :
une première indication de rétroaction si le système de distribution d'aérosol (100) détermine que le composant de distribution d'aérosol (104) est valide pour une utilisation avec le dispositif de distribution d'aérosol (102) ; et
une seconde indication de rétroaction si le système de distribution d'aérosol (100) détermine que le composant de distribution d'aérosol (104) n'est pas valide pour une utilisation avec le dispositif de distribution d'aérosol (102),
la seconde indication de rétroaction étant différente de la première indication de rétroaction.

11. Système de distribution d'aérosol (100) selon la revendication 10, dans lequel l'élément de rétroaction (160) du composant de distribution d'aérosol (104) comprend un élément de rétroaction visuelle (160),
la première indication de rétroaction est l'élément de rétroaction visuelle (160) émettant une lumière d'une première couleur, et la seconde indication de rétroaction est l'élément de rétroaction visuelle (160) émettant une lumière d'une seconde couleur, la seconde couleur étant différente de la première couleur.

12. Système de distribution d'aérosol (100) selon l'une quelconque des revendications précédentes, dans lequel le système de distribution d'aérosol (100) est configuré pour déterminer si le composant de distribution d'aérosol (104) est valide ou non pour une utilisation avec le dispositif de distribution d'aérosol (102), et l'élément de rétroaction (160) est configuré pour fournir une rétroaction à l'utilisateur sur la base de la détermination que le composant de distribution d'aérosol (104) est valide ou non pour une utilisation avec le dispositif de distribution d'aérosol (102), lorsque le composant de distribution d'aérosol (104) est mis en prise avec le dispositif de distribution d'aérosol (102).

13. Système de distribution d'aérosol (100) selon l'une quelconque des revendications précédentes, dans lequel le système de distribution d'aérosol (100) est configuré pour, s'il est déterminé à partir des informations d'état de validité, que le composant de distribution d'aérosol (104) est invalide pour une utilisation avec le dispositif de distribution d'aérosol (102), empêcher une utilisation du composant de distribution d'aérosol (104) avec le dispositif de distribution d'aérosol (102).

14. Système de distribution d'aérosol (100) selon l'une quelconque des revendications précédentes, dans lequel le composant de distribution d'aérosol (104) comprend un dispositif de mémoire configuré pour stocker les informations d'état de validité associées au composant de distribution d'aérosol (104), et dans lequel le dispositif de distribution d'aérosol (102) est configuré pour obtenir les informations d'état de validité associées au composant de distribution d'aérosol (104) et stockées dans le dispositif de mémoire du composant de distribution d'aérosol (104) via l'interface entre le composant de distribution d'aérosol (104) et le dispositif de distribution d'aérosol (102).

15. Procédé mis en œuvre par un système de distribution d'aérosol (100), dans lequel le système de distribution d'aérosol inclut un dispositif de distribution d'aérosol (102) et un composant de distribution d'aérosol (104) pouvant venir en prise avec le dispositif de distribution d'aérosol (102) au niveau d'une interface entre eux, le composant de distribution d'aérosol (104) étant configuré pour loger un précurseur d'aérosol, et le composant de distribution d'aérosol (104) comprenant un élément de rétroaction (160), et dans lequel le composant de distribution d'aérosol (104) est associé à des informations d'état de validité à partir desquelles il est possible de déterminer si le composant de distribution d'aérosol (104) est valide ou non pour une utilisation avec le dispositif de distribution d'aérosol (102), le procédé incluant les étapes consistant à :
déterminer à partir des informations d'état de validité si le composant de distribution d'aérosol (104) est valide ou non pour une utilisation avec le dispositif de distribution d'aérosol (102) ; et
fournir, par l'intermédiaire de l'élément de rétroaction du composant de distribution d'aérosol (104), une rétroaction à un utilisateur sur la base de la détermination de la validité ou non du composant de distribution d'aérosol (104) pour une utilisation avec le système de distribution d'aérosol.
